# FASCICULE DE BREVET EUROPEEN

(11) **EP 1 827 495 B1**
(45) Date de publication et mention de la délivrance du brevet: **15.12.2010**
(21) Numéro de dépôt: 05849336.2
(22) Date de dépôt: 16.12.2005
(51) Int. Cl.: A61K 45/06, A61K 31/4985, A61P 29/00

(54) **ASSOCIATIONS ANTI-DOULEUR COMPRENANT UN DERIVE DE DIHYDROIMIDAZOPYRAZINE**
SCHMERZLINDERNDE ASSOZIATION MIT EINEM DIHYDROIMIDAZOPYRAZIN-DERIVAT
PAINKILLING ASSOCIATION COMPRISING A DIHYDROIMIDAZOPYRAZINE DERIVATIVE

(30) Priorité: 17.12.2004 FR 0413453
(43) Date de publication de la demande: 05.09.2007
(73) Titulaire: IPSEN PHARMA, 92100 Boulogne-Billancourt (FR)
(72) Inventeur: AUGUET, Michel, F-91120 Palaiseau (FR); FAVRE, Christine, F-91530 Saint Maurice Montcouronne (FR); PREVOST, Grégoire, F-92160 Antony (FR); CHABRIER DE LASSAUNIERE, Pierre-Etienne, F-75016 Paris (FR)
(74) Mandataire: Audonnet, Nathalie
(86) Numéro de dépôt international: PCT/FR2005/003162
(87) Numéro de publication internationale: WO 2006/067312

(56) Documents cités:
- WO-A-00/02881
- WO-A-01/49322
- WO-A-97/30053
- WO-A-2004/110415
- WO-A-2005/000852
- KASPRZYK PHILIP G ET AL: "G-protein signaling inhibitor, BIM-46174, improves the efficacy of anticancer drugs" PROCEEDINGS OF THE AMERICAN ASSOCIATION FOR CANCER RESEARCH ANNUAL MEETING, vol. 43, mars 2002 (2002-03), pages 75-76, XP008077341 & 93RD ANNUAL MEETING OF THE AMERICAN ASSOCIATION FOR CANCER RESEARCH; SAN FRANCISCO, CALIFORNIA, USA; APRIL 06-10, 2002 ISSN: 0197-016X

## Description

La présente invention concerne des associations antidouleur comprenant un dérivé de dihydroimidazopyrazine, à savoir le (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine ou un de ses sels pharmaceutiquement acceptables.

La douleur reste encore aujourd'hui une pathologie difficile à soulager ou à guérir. L'usage des composés actuellement disponibles qui permettent de réduire la douleur de manière satisfaisante est souvent associé à des effets secondaires indésirables (sédations, accoutumance, hyperalgésie, risque d'ulcères). Pour réduire ces risques d'effets secondaires, la combinaison de plusieurs agents anti-douleur agissant par des mécanismes différents est souvent utilisée. Cela permet d'améliorer le traitement de la douleur tout en réduisant les risques d'effets secondaires indésirables en utilisant des doses réduites de chacun des agents.

Le (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine a déjà été décrite par la demanderesse en tant qu'agent anti-cancéreux.

La morphine, aujourd'hui bien connue pour ses effets anti-douleur, a quant à elle été isolée au tout début du XIX^{e} siècle par un pharmacien allemand, Friedrich Sertürner, à partir de l'opium dont elle est le constituant principal.

La Demanderesse vient à présent de découvrir que l'association de (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine et de morphine, présente un effet synergique puissant dans le traitement de la douleur au point de pouvoir réduire considérablement les doses de morphine ou d'analogue ou dérivé de la morphine administrées au patient tout en conservant un effet analgésique équivalent. En effet, ces deux principes actifs administrés à des doses subactives (c'est-à-dire à des doses qui ne produisent pas par elles-mêmes d'effet thérapeutique), produisent, lorsqu'ils sont associés, un effet thérapeutique hautement significatif.

La présente invention concerne un produit comprenant du (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine ou un de ses sels pharmaceutiquement acceptables en association avec un agent analgésique choisi parmi les ligands des récepteurs opiacés ou leurs sels, comme par exemple la morphine, les inhibiteurs des canaux sodiques, les anti-inflammatoires non stéroïdiens (AINS), les inhibiteurs du système glutamatergique, les antidépresseurs tricycliques, les agonistes alpha 2 adrénergiques, les cannabinoïdes et les dérivés gabaergiques pour une utilisation thérapeutique simultanée, séparée ou étalée dans le temps dans le traitement ou la prévention de la douleur.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J Pharm. (1986), 33, 201-217.

Par utilisation thérapeutique simultanée, on entend dans la présente demande une administration de plusieurs principes actifs par la même voie et au même moment. Par utilisation séparée, on entend notamment une administration de plusieurs principes actifs sensiblement au même moment par des voies différentes. Par utilisation thérapeutique étalée dans le temps, on entend une administration de plusieurs principes actifs à des moments différents et notamment un mode d'administration selon lequel l'ensemble de l'administration de l'un des principes actifs est effectué avant que l'administration de l'autre ou des autres ne commence. On peut ainsi administrer l'un des principes actifs pendant plusieurs mois avant d'administrer l'autre ou les autres principes actifs. Il n'y a pas de traitement simultané dans ce cas.

Par ligands des récepteurs opiacés ou leurs sels, on entend les substances choisies parmi la naloxone, la naltrexone, la nalorphine, le fentanyl, l'alfentanil, la codéine, la dihydrocodéine, l'hydrocodone, l'oxycodone, l'hydromorphone, la péthidine, le rémifentanyl, le sufentanil, le dextropropoxyphène, le tramadol, la buprénorphine, la nalbuphine, la morphine, le sulfate de morphine, le chlorhydrate d'hydromorphone et le sulfate de morphine enrobé.

Par « inhibiteurs des canaux sodiques », on entend notamment les composés suivants (éventuellement sous forme de sels pharmaceutiquement acceptables) :
❖ la carbamazépine ;
❖ la lidocaïne ;
❖ la tétracaïne ;
❖ la bupivacaïne ;
❖ la procaïne ;
❖ la mépivacaïne ;
❖ la dibucaïne ;
❖ la lamotrigine ;
❖ la mexilitine ;
❖ le riluzole ; ou
❖ le 2-(4-[1,1'-biphényl]-4-yl-1H-imidazol-2-yl)éthylcarbamate de butyle (composé ICS).

Par « agents anti-inflammatoires non stéroïdiens (AINS) », on entend notamment les composés suivants (éventuellement sous forme de sels pharmaceutiquement acceptables) :
❖ l'acide acétylsalicylique et ses dérivés ;
❖ les AINS indoliques et dérivés comme par exemple l'indométacine et le sulindac ;
❖ les AINS arylcarbocycliques comme l'acide tiaprofénique, l'alminoprofène, le diclofénac, l'étodolac, le flurbiprofène, l'ibuprofène, le kétoprofène, la nabumétone ou le naproxène ;
❖ les AINS dérivés oxicam comme le meloxicam, le piroxicam ou le ténoxicam ;
❖ le morniflumate ;
❖ la butazolidine ;
❖ les inhibiteurs sélectifs de la cyclooxygénase-2 (COX-2) comme le célécoxib ou le rofécoxib, le lumiracoxib, le valdécoxib, le déracoxib, parécoxib, l'étoricoxib et la nimésulide ; ou
❖ l'acétaminophène (paracétamol) et le dipyrone.

Par « inhibiteurs du système glutamatergique », on entend notamment les composés suivants (éventuellement sous forme de sels pharmaceutiquement acceptables) :
❖ la kétamine ;
❖ l'amantadine ;
❖ la mémentine ; ou
❖ le dextrométorphane.

Par « antidépresseurs tricycliques », on entend notamment les composés suivants (éventuellement sous forme de sels pharmaceutiquement acceptables) :
❖ la clomipramine ;
❖ l'amoxapine ;
❖ l'amitriptyline ;
❖ la désipramine ;
❖ le chlorhydrate de dothiépine ;
❖ la doxépine ;
❖ l'imipramine ;
❖ la nortriptyline ;
❖ la protryptiline ;
❖ la trimipramine
❖ la mirtazapine ;
❖ la duloxétine ; ou
❖ le milnacipran.

Par « agonistes alpha 2 adrénergiques », on entend notamment les composés suivants (éventuellement sous forme de sels pharmaceutiquement acceptables) :
❖ la clonidine ;
❖ la dexmédétomidine ;
❖ le mivazérol ;
❖ la loféxidine ;
❖ la guanfacine ; ou
❖ l'acétate de guanabenz.

Par « cannabinoïdes », on entend notamment, sans que cela soit limitatif :
- le CP55940: ((-)-cis-3-[2-Hydroxy-4-(1,1-dimethylheptyl)phenyl]-trans-4(3-hydroxypropyl)cyclohexanol),
- l'AM1241 : (3-(2-Iodo-5-nitrobenzoyl)-1-(1-methyl-2-piperidinylmethyl)-1H-indole,
- le WIN55212-2: (R)-(+)-[2,3-Dihydro-5-methyl-3[(morpholinyl)-methyl]pyrrolol[1,2,3-de]-1,4-benzoxazinyl]-(1-naphthalenyl)methadone mesylate ou ((R)-(+)-[2,3-Dihydro-5-methyl-3-(4-morpholinylmethyl)pyrrolo[1,2,3-de]-1,4-benzoxazin-6-yl]-1-naphthalenylmethadone),
- le JWH-133 : 3-(1',1'-Dimethylbityl)-1-deoxy-Δ⁸-tetrahydrocannabinol ;3-(1',1'-Dimethylbutyl)-1-deoxy-Δ⁸-THC,
- le JWH-051 : (2-Methyl-1-propyl-1H-indol-3-yl)-1-naphthalenylmethanone, ou
- le dronabinol.

Par « dérivés gabaergiques », on entend notamment les composés suivants (éventuellement sous forme de sels pharmaceutiquement acceptables) :
❖ la gabapentine ;
❖ le baclofène ; ou
❖ la prégabaline.

Par « douleur », il faut entendre dans la présente demande « toute expérience désagréable émotionnelle et sensorielle associée à un dommage tissulaire présent ou potentiel ou décrite par le patient en de tels termes ».

Une variante préférée de l'invention concerne un produit comprenant du (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine ou un de ses sels pharmaceutiquement acceptables en association avec de la morphine pour une utilisation thérapeutique simultanée, séparée ou étalée dans le temps dans le traitement ou la prévention de la douleur.

En particulier, l'invention aura pour objet un produit comprenant du (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine ou un de ses sels pharmaceutiquement acceptables en association avec de la morphine pour une utilisation thérapeutique simultanée, séparée ou étalée dans le temps dans le traitement ou la prévention de la douleur.

L'invention offre aussi un produit comprenant du (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine ou un de ses sels pharmaceutiquement acceptables en association avec un agent analgésique choisi parmi les inhibiteurs des canaux sodiques, les anti-inflammatoires non stéroïdiens (AINS), les inhibiteurs du système glutamatergique, les antidépresseurs tricycliques et les dérivés gabaergiques pour une utilisation thérapeutique simultanée, séparée ou étalée dans le temps dans le traitement ou la prévention de la douleur.

Parmi les douleurs pouvant être traitées par un produit selon l'invention, on pourra citer notamment :
❖ les douleurs liées à un cancer (particulièrement préférées dans la mesure où le (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine est également un agent anti-cancéreux) ;
❖ les douleurs liées à des maladies chroniques autres qu'un cancer telles que les douleurs liées à des maladies virales ou rétrovirales (par exemple les douleurs liées ou consécutives au Syndrome Immuno-Déficitaire Acquis (SIDA) ou les douleurs liées au zona) ou les douleurs liées à des neuropathies diabétiques ;
❖ les douleurs neuropathiques telles que la névralgie du trijumeau, les névralgies glosso-pharyngiennes, les douleurs liées à des radiculopathies, à des neuropathies diabétique ou aux agents anti-cancéreux, les douleurs inflammatoires et les douleurs liées à des neuropathies secondaires à des infiltrations métastasiques ;
❖ l'adiposis dolorosa ;
❖ les douleurs liées aux brûlures ;
❖ la migraine ;
❖ les douleurs pré- et post-opératoires ;
❖ les douleurs chroniques;
❖ les douleurs chroniques, la fibromyalgie, l'algoneurodystrophie ou syndrome douloureux régional complexe (« *Complex Regional Pain Syndrome »* en anglais) ;
❖ les douleurs centrales consécutives aux accidents cérébraux vasculaires, aux lésions thalamiques ou à la sclérose en plaques

L'administration d'un médicament selon l'invention pourra se faire par voie topique, par voie orale, par voie parentérale, par injection intramusculaire, par injection sous-cutanée, par injection intra-veineuse, etc. ou par une combinaison de ces voies.

La dose d'un produit selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A moins qu'ils ne soient définis d'une autre manière, tous les termes techniques et scientifiques utilisés ici ont la même signification que celle couramment comprise par un spécialiste ordinaire du domaine auquel appartient cette invention.
La figure 1 présente l'effet du composé **(1)** suite à l'injection par voie intra-veineuse dans le modèle d'hyperalgésie induite par la carragénine.
La figure 2 présente l'effet de la morphine suite à l'injection par voie intra-péritonéale dans le modèle d'hyperalgésie induite par la carragénine.
Les figures 3 et 4 présentent l'effet de l'association du composé **(1)** par voie intra-veineuse et de la morphine par voie intra-péritonéale dans le modèle d'hyperalgésie induite par la carragénine.
La figure 5 présente l'effet du fentanyl par voie intra-péritonéale seul et en association avec le composé **(1)** par voie intra-veineuse dans le modèle d'hyperalgésie induite par la carragénine.
La figure 6 présente l'effet de la lidocaïne par voie intra-péritonéale seule et en association avec le composé **(1)** suite à l'injection par voie intra-veineuse dans le modèle d'hyperalgésie induite par la carragénine.
La figure 7 présente l'effet d'un inhibiteur des canaux sodiques: (composé ICS) par voie intra-péritonéale et en association avec le composé **(1)** suite à l'injection par voie intra-veineuse dans le modèle d'hyperalgésie induite par la carragénine.
La figure 8 présente l'effet de l'ibuprofene par voie intra-péritonéale seul et en association avec le composé **(1)** suite à l'injection par voie intra-veineuse dans le modèle d'hyperalgésie induite par la carragénine.
La figure 9 présente l'effet de la kétamine par voie intra-péritonéale seul et en association avec le composé **(1)** suite à l'injection par voie intra-veineuse dans le modèle d'hyperalgésie induite par la carragénine.
La figure 10 présente l'effet d'un agoniste des récepteurs cannabinoïdes (CP55940) par voie intra-péritonéale et en association avec le composé **(1)** suite à l'injection par voie intra-veineuse dans le modèle d'hyperalgésie induite par la carragénine.
La figure 11 présente l'effet d'un ligand des récepteurs opiacés seul (naloxone) par voie sous-cutanée et en association avec le composé **(1)** suite à l'injection par voie intra-veineuse dans le modèle d'hyperalgésie induite par la carragénine.

Les exemples suivants sont présentés pour illustrer les procédures ci-dessus.

### Etude pharmacologique des produits de l'invention

L'activité des composés de l'invention a été évaluée in vivo sur un modèle d'hyperalgésie induite par la carragénine sur la patte de rat.

Des rats mâles Sprague Dawley (Charles River) de 200 à 240 g le jour de l'expérience sont laissés en stabulation 5 à 8 jours dans les conditions d'animalerie et sont mis à jeun sur grilles 18 h avant et pendant l'expérience. Les groupes sont constitués d'au moins 6 animaux. Les produits sont administrés par voie intra-péritonéale (i.p., 2 ml/kg) ou intra-veineuse (i.v., 1 ml/kg), 2 h 30 après l'injection de carragénine. La carragénine 2% a été injectée par voie sous-plantaire dans la patte arrière droite des rats. Le seuil nociceptif a été évalué en mesurant le retrait de la patte du rat suscité par un stimulus mécanique appliqué à l'aide d'un analgésie-mètre (test de Randall-Selitto). Les mesures ont été faites juste avant l'injection de carragénine (t = - 2 h 30) et 30 min, 2h30 et 4h après l'injection des produits à tester (effectuée à t = 0). L'efficacité des produits est évaluée par leur capacité à réduire de manière significative l'hyperalgésie induite par la carragénine. Cette efficacité sera statistiquement déterminée par un test d'analyse de variance (une voie) et/ou d'un test de Dunnett (deux voies).

Dans les exemples ci-après, on désigne par « Composé 1 » le (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine (un inhibiteur du signal médié par les protéines G hétérotrimériques).

### Exemple 1 : Effet du Composé 1 sur l'hyperalgésie induite par la carragénine :

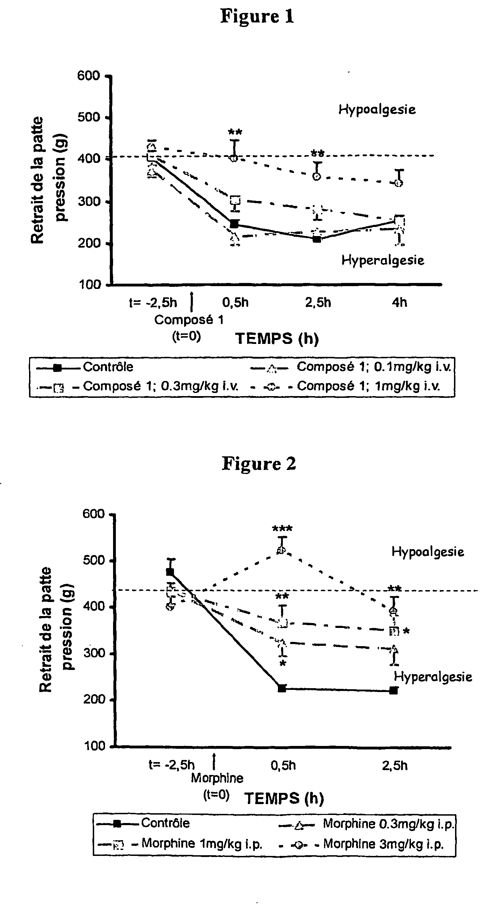

Les résultats obtenus en utilisant différentes doses du Composé 1 dans le modèle d'hyperalgésie induite par la carragénine sur la patte de rat décrit ci-dessus sont reportés dans la Figure 1.

L'activité analgésique du composé 1 est démontrée dans le test d'hyperalgésie induite par la carragénine. A partir de la dose de 1 mg/kg (i.v.) le seuil de douleur suite à un stimulus mécanique appliqué sur les pattes des rats, est significativement réduit.

### Exemple 2 : Effet de la morphine sur l'hyperalgésie induite par la carragénine :

Les résultats obtenus en utilisant différentes doses de la morphine dans le modèle d'hyperalgésie induite par la carragénine sur la patte de rat décrit ci-dessus sont reportés dans la Figure 2.

L'activité analgésique de la morphine est démontrée dans le test d'hyperalgésie induite par la carragénine. A partir de la dose de 1 mg/kg (i.p.) le seuil de douleur suite à un stimulus mécanique appliqué sur les pattes des rats, est significativement réduit.

### Exemple 3 : Effet de l'association du Composé 1 et de la morphine sur l'hyperalgésie induite par la carragénine :

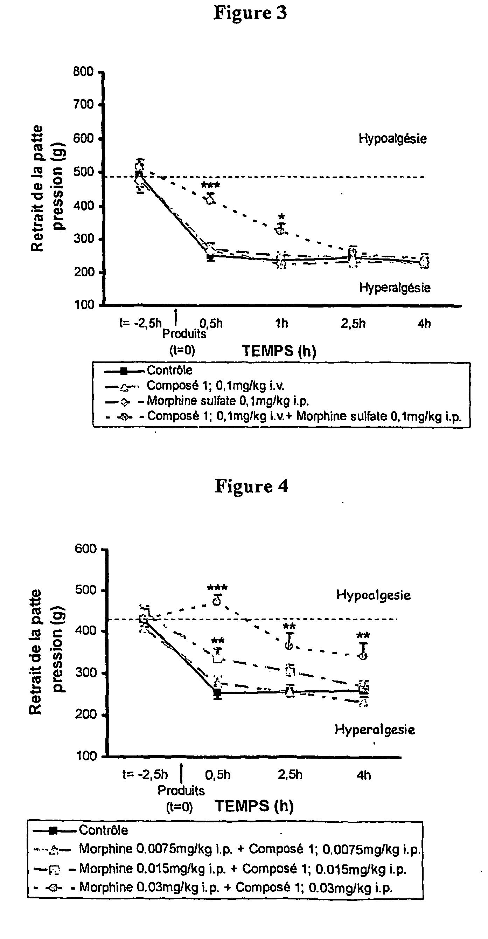

Les résultats obtenus en utilisant différentes associations de Composé 1 et de morphine dans le modèle d'hyperalgésie induite par la carragénine sur la patte de rat décrit ci dessus sont reportés dans les Figure 3 et 4.

L'activité analgésique de l'association du composé 1 + morphine est démontrée dans le même modèle que précédemment. L'effet analgésique de cette combinaison est supérieur en puissance et plus durable que les effets de chacun des composées utilisés seuls (autrement dit, on observe un effet synergique). Une dose de 0,015 mg/kg (i.p.) de morphine dans le cadre d'une telle association s'avère alors efficace.

En comparant avec les résultats des exemples 1 et 2, l'on constate donc que l'administration de Composé 1 et de morphine ont un effet analgésique synergique lorsqu'ils sont associés (figure 4). En effet, l'utilisation de Composé 1 permet de réduire d'au moins un facteur 30-50 les doses de morphine nécessaires pour obtenir un effet équivalent.

### Exemple 4 : Effet de l'association du Composé 1 et du fentanyl sur l'hyperalgésie induite par la carragénine :

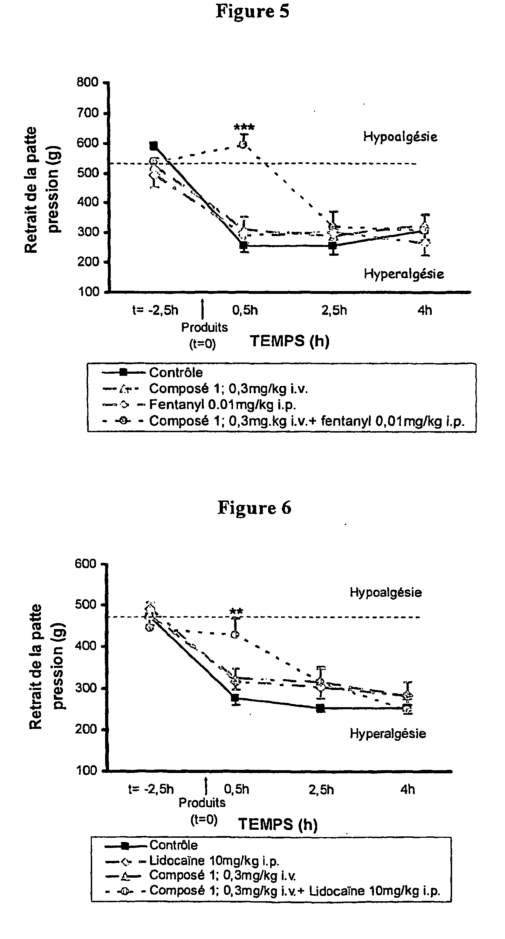

Les résultats obtenus en utilisant différentes associations de Composé 1 et de fentanyl dans le modèle d'hyperalgésie induite par la carragénine sur la patte de rat décrit ci dessus sont reportés dans la Figure 5.

On observe une synergie dès que le fentanyl est associé au composé (1). En effet au temps 0,5h, l'association permet d'augmenter le seuil de douleur toléré par le rat sur sa patte enflammée lorsque l'on applique une pression croissante. Le seuil de douleur qui était de environ 250 - 300 g/mm² pour le contrôle ou le composé (1) seul, est sous l'effet de l'association de 600 g/mm².

### Exemple 5: Effet de l'association du Composé 1 et de la lidocaïne sur l'hyperalgésie induite par la carragénine :

Les résultats obtenus en utilisant différentes associations de Composé 1 et de lidocaïne dans le modèle d'hyperalgésie induite par la carragénine sur la patte de rat décrit ci dessus sont reportés dans la Figure 6.

On observe une synergie dès que la lidocaïne est associé au composé (1). En effet au temps 0,5h, l'association permet d'augmenter le seuil de douleur toléré par le rat sur sa patte enflammée lorsque l'on applique une pression croissante. Le seuil de douleur qui était de environ 280 - 320 g/mm² pour le contrôle ou le composé (1) seul, est sous l'effet de l'association de 430 g/mm².

### Exemple 6 : Effet de l'association du Composé 1 et d'un inhibiteur des canaux sodiques (composé ICS) sur l'hyperalgésie induite par la carragénine :

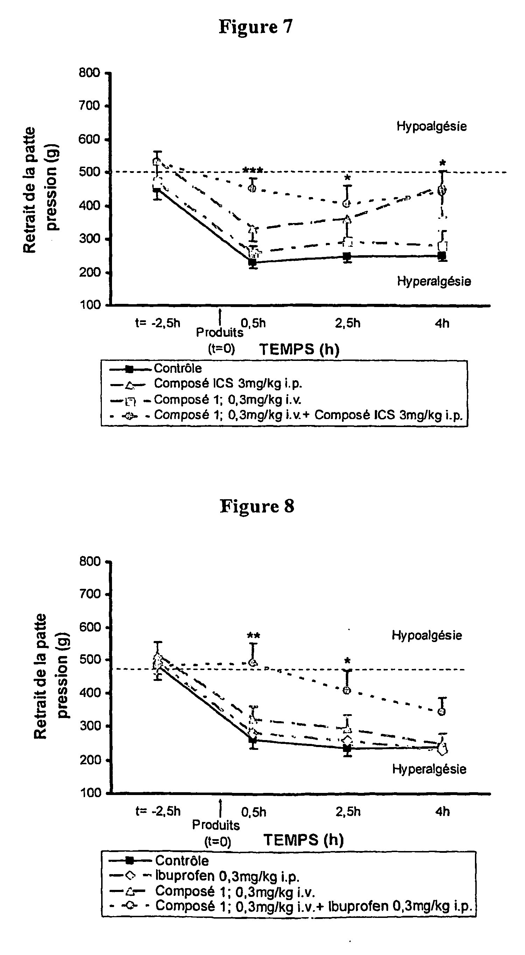

Les résultats obtenus en utilisant différentes associations de Composé 1 et d'un inhibiteur des canaux sodiques dans le modèle d'hyperalgésie induite par la carragénine sur la patte de rat décrit ci dessus sont reportés dans la Figure 7.

Le composé inhibiteur des canaux sodiques utilisé est le butyl 2-[4-(1,1'-biphenyl-4-yl)-1H-imidazol-2-yl]ethylcarbamate (dénommé composé ICS).

On observe une synergie dès que le butyl 2-[4-(1,1'-biphenyl-4-yl)-1H-imidazol-2-yl]ethylcarbamate est associé au composé (1). En effet au temps 0,5h, l'association permet d'augmenter le seuil de douleur toléré par le rat sur sa patte enflammée lorsque l'on applique une pression croissante. Le seuil de douleur qui était de environ 250 g/mm² pour le contrôle ou le composé (1) seul, est sous l'effet de l'association de 450 g/mm².

### Exemple 7 : Effet de l'association du Composé 1 et de l'ibuprofen sur l'hyperalgésie induite par la carragénine :

Les résultats obtenus en utilisant différentes associations de Composé 1 et de l'ibuprofen dans le modèle d'hyperalgésie induite par la carragénine sur la patte de rat décrit ci dessus sont reportés dans la Figure 8.

On observe une synergie dès que l'ibuprofen est associé au composé (1). En effet au temps 0,5h, l'association permet d'augmenter le seuil de douleur toléré par le rat sur sa patte enflammée lorsque l'on applique une pression croissante. Le seuil de douleur qui était de environ 260 - 320 g/mm² pour le contrôle ou le composé (1) seul, est sous l'effet de l'association de 500 g/mm².

### Exemple 8 : Effet de l'association du Composé 1 et de la kétamine sur l'hyperalgésie induite par la carragénine :

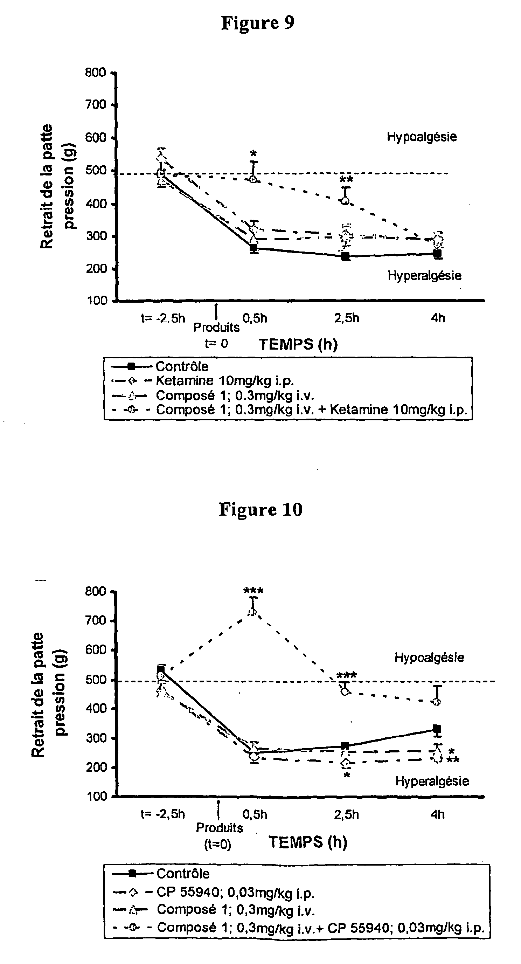

Les résultats obtenus en utilisant différentes associations de Composé 1 et de kétamine dans le modèle d'hyperalgésie induite par la carragénine sur la patte de rat décrit ci dessus sont reportés dans la Figure 9.

On observe une synergie dès que la kétamine est associé au composé (1). En effet au temps 0,5h, l'association permet d'augmenter le seuil de douleur toléré par le rat sur sa patte enflammée lorsque l'on applique une pression croissante. Le seuil de douleur qui était de environ 260 - 290 g/mm² pour le contrôle ou le composé (1) seul, est sous l'effet de l'association de 480 g/mm².

### Exemple 9 : Effet de l'association du Composé 1 et d'un agoniste des récepteurs cannabinoïdes (CP55940) sur l'hyperalgésie induite par la carragénine :

Les résultats obtenus en utilisant différentes associations de Composé 1 et d'un agoniste des récepteurs cannabinoïdes dans le modèle d'hyperalgésie induite par la carragénine sur la patte de rat décrit ci dessus sont reportés dans la Figure 10.

On observe une synergie dès que le CP 55940 est associé au composé (1). En effet au temps 0,5h, l'association permet d'augmenter le seuil de douleur toléré par le rat sur sa patte enflammée lorsque l'on applique une pression croissante. Le seuil de douleur qui était de environ 260 g/mm² pour le contrôle ou le composé (1) seul, est sous l'effet de l'association de 730 g/mm².

### Exemple 10 : Effet de l'association du Composé 1 et d'un ligand des récepteurs opiacés sur l'hyperalgésie induite par la carragénine :

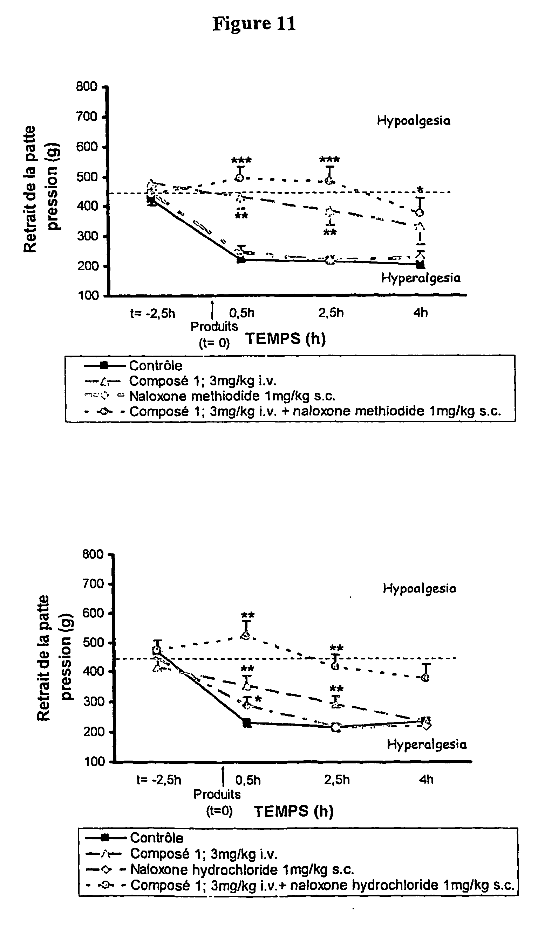

Les résultats obtenus en utilisant différentes associations de Composé 1 et d'un ligand des récepteurs opiacés dans le modèle d'hyperalgésie induite par la carragénine sur la patte de rat décrit ci dessus sont reportés dans la Figure 11.

Le composé ligand des récepteurs opiacés utilisé est la naloxone.

On observe une potentialisation dès que la naloxone est associé au composé (1). En effet au temps 0,5h, l'association permet d'augmenter le seuil de douleur toléré par le rat sur sa patte enflammée lorsque l'on applique une pression croissante. Le seuil de douleur qui était de environ 220 g/mm² pour le contrôle et de 430 pour le composé (1) seul (3mg /kg i.v.), est sous l'effet de l'association de 500 g/mm².

L'exemple 10 ne fait pas partie de l'invention revendiquée.

## Revendications

1. Produit comprenant du (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine ou un de ses sels pharmaceutiquement acceptables en association avec un agent analgésique choisi parmi le fentanyl, l'alfentanil, la codéine, la dihydrocodéine, l'hydrocodone, l'oxycodone, l'hydromorphone, la péthidine, le rémifentanyl, le sufentanil, le dextropropoxyphène, le tramadol, la buprénorphine, la nalbuphine, la morphine, le sulfate de morphine, le chlorhydrate d'hydromorphone et du sulfate de morphine enrobé, la morphine, les inhibiteurs des canaux sodiques, les anti-inflammatoires non stéroïdiens (AINS), les inhibiteurs du système glutamatergique, les antidépresseurs tricycliques et les dérivés gabaergiques ces derniers étant choisis parmi la gabapentine, le baclofène ou la prégabaline, pour une utilisation thérapeutique simultanée, séparée ou étalée dans le temps dans le traitement ou la prévention de la douleur.

2. Produit selon la revendication 1, **caractérisé en ce que** le (1*R*)-1-[({(2*R*)-2-amino-3-[(8*S*)-8-(cyclohexylméthyl)-2-phényl-5,6-dihydroimidazo[1,2-α]pyrazin-7(8*H*)-yl]-3-oxopropyl}dithio)méthyl]-2-[(8*S*)-8-(cyclohexylméthyl)-2-phényl-5,6-dihydroimidazo [1,2-α]pyrazin-7(8*H*)-yl]-2-oxoéthylamine ou un de ses sels pharmaceutiquement acceptables est associé à la morphine pour une utilisation thérapeutique simultanée, séparée ou étalée dans le temps dans le traitement ou la prévention de la douleur.

3. Produit selon la revendication 2, **caractérisé en ce qu'**il consiste en l'association de (1*R*)-1-[({(2*R*)-2-amino-3-[(8*S*)-8-(cyclohexylméthyl)-2-phényl-5,6-dihydroimidazo [1,2-α]pyrazin-7(8*H*)-yl]-3-oxopropyl}dithio)méthyl]-2-[(8*S*)-8-(cyclohexylméthyl)-2-phényl-5,6-dihydroimidazo[1,2-α]pyrazin-7(8*H*)-yl]-2-oxoéthylamine ou d'un de ses sels pharmaceutiquement acceptables avec de la morphine pour une utilisation thérapeutique simultanée, séparée ou étalée dans le temps dans le traitement ou la prévention de la douleur.

4. Produit selon l'une des revendications 1 à 3, **caractérisé en ce qu'**il est destiné à une utilisation simultanée.

5. Produit selon l'une des revendications 1 à 3, **caractérisé en ce qu'**il est destiné à une utilisation séparée.

6. Produit selon l'une des revendications 1 à 3, **caractérisé en ce qu'**il est destiné à une utilisation étalée dans le temps.

7. Produit selon l'une des revendications 1 à 6 **caractérisé en ce que** la douleur destinée à être traitée est choisie parmi les douleurs liées à un cancer, les douleurs liées à des maladies chroniques autres qu'un cancer, les douleurs neuropathiques, les douleurs liées à des radiculopathies, l'adiposis dolorosa, les douleurs liées aux brûlures, la migraine, les douleurs pré- et post-opératoires, les douleurs chroniques, la fibromyalgie, l'algoneurodystrophie ou syndrome douloureux régional complexe et les douleurs centrales consécutives aux accidents cérébraux vasculaires, aux lésions thalamiques ou à la sclérose en plaques.

8. Produit selon la revendication 7, **caractérisé en ce que** la douleur destinée à être traitée est liée à un cancer.

9. Produit selon la revendication 7, **caractérisé en ce que** la douleur destinée à être traitée est liée à une maladie chronique autre qu'un cancer.

## Claims

1. Product comprising (1*R*)-1-[({(2*R*)-2-amino-3-[(8*S*)-8-(cyclohexylmethyl)-2-phenyl-5,6-dihydroimidazo[1,2-α]pyrazin-7(8*H*)-yl]-3-oxopropyl}dithio)methyl]-2-[(8*S*)-8-(cyclohexylmethyl)-2-phenyl-5,6-dihydroimidazo[1,2-α]pyrazin-7(8*H*)-yl]-2-oxoethylamine or one of its pharmaceutically acceptable salts in combination with an analgesic agent chosen from fentanyl, alfentanil, codeine, dihydrocodeine, hydrocodone, oxycodone, hydromorphone, pethidine, remifentanyl, sufentanyl, dextropropoxyphene, tramadol, buprenorphine, nalbuphine, morphine, morphine sulphate, hydromorphone hydrochloride and coated morphine sulphate, morphine, sodium channel inhibitors, non-steroidal anti-inflammatories (NSAID), glutamatergic system inhibitors, tricyclic antidepressants and gabaergic derivatives, these gabaergic derivatives being chosen from gabapentin, baclofen or pregabalin, for a therapeutic use which is simultaneous, separate or spread out over time for the treatment or prevention of pain.

2. Product according to claim 1, **characterized in that** (1*R*)-1-[({(2*R*)-2-amino-3-[(8*S*)-8-(cyclohexylmethyl)-2-phenyl-5,6-dihydroimidazo[1,2-α]pyrazin-7(8*H*)-yl]-3-oxopropyl}dithio)methyl]-2-[(8*S*)-8-(cyclohexylmethyl)-2-phenyl-5,6-dihydroimidazo [1,2-α]pyrazin-7(8*H*)-yl]-2-oxoethylamine or one of its pharmaceutically acceptable salts is combined with morphine for a therapeutic use which is simultaneous, separate or spread out over time for the treatment or prevention of pain.

3. Product according to claim 2, **characterized in that** it consists of the combination of (1*R*)-1-[({(2*R*)-2-amino-3-[(8*S*)-8-(cyclohexylmethyl)-2-phenyl-5,6-dihydroimidazo [1,2-α]pyrazin-7(8*H*)-yl]-3-oxopropyl}dithio)methyl]-2-[(8*S*)-8-(cyclohexylmethyl)-2-phenyl-5,6-dihydroimidazo[1,2-α]pyrazin-7(8*H*)-yl]-2-oxoethylamine or one of its pharmaceutically acceptable salts with morphine for a therapeutic use which is simultaneous, separate or spread out over time for the treatment or prevention of pain.

4. Product according to one of claims 1 to 3, **characterized in that** it is intended for simultaneous use.

5. Product according to one of claims 1 to 3, **characterized in that** it is intended for separate use.

6. Product according to one of claims 1 to 3, **characterized in that** it is intended for use spread out over time.

7. Product according to one of claims 1 to 6, **characterized in that** the pain to be treated is chosen from the types of pain associated with a cancer, pain associated with chronic diseases other than a cancer, neuropathic pain, pain associated with radiculopathies, adiposis dolorosa, pain associated with bums, migraine, pre- and post-operative pain, chronic inflammatory pain, fibromyalgia, algoneurodystrophy or complex regional pain syndrome and central pain following cerebral vascular accidents, thalamic lesions or multiple sclerosis.

8. Product according to claim 7, **characterized in that** the pain intended to be treated is associated with a cancer.

9. Product according to claim 7, **characterized in that** the pain intended to be treated is associated with a chronic disease other than a cancer.

## Patentansprüche

1. Produkt, das (1R)-1-[({(2R)-2-Amino-3-[(8S)-8-(cyclohexylmethyl)-2-phenyl-5,6-dihydroimidazo[1,2-a]pyrazin-7(8H)-yl]-3-oxopropyl}dithio)methyl]-2-[(8S)-8-(cyclohexylmethyl)-2-phenyl-5,6-dihydroimidazo[1,2-a]pyrazin-7(8H)-yl]-2-oxoethylamin oder eines seiner pharmazeutisch verträglichen Salze in Kombination mit einem Analgetikum, ausgewählt aus Fentanyl, Alfentanil, Codein, Dihydrocodein, Hydrocodon, Oxycodon, Hydromorphon, Pethidin, Remifentanyl, Sufentanil, Dextropropoxyphen, Tramadol, Euprenorphin, Nalhuphin, Morphin, Morphinsulfat, Hydromorphon-Hydrochlorid und überzogenem Morphinsulfat, Morphin, Inhibitoren der Natriumkanäle, nicht-steroidalen Antirheumatika (NSAR), Inhibitoren des glutamatergen Systems, tricyclischen Antidepressiva und gabaergischen Derivaten, wobei diese letztgenannten unter Gabapentin, Baclofen und Pregabalin ausgewählt sind, umfasst, für eine gleichzeitige, getrennte oder sich über einen bestimmten Zeitraum erstreckende therapeutische Verwendung bei der Behandlung oder Prävention von Schmerz.

2. Produkt nach Anspruch 1, **dadurch gekennzeichnet, dass** (1R)-1-[({(2R)-2-Amino-3-1(8S)-8-(cyclohexylmethyl)-2-phenyl-5,6-dihydroimidazo[1,2-α]pyrazin-7(8H)-yl]-3-oxopropyl}dithio)methyl]-2-[(8S)-8-(cyclohexylmethyl)-2-phenyl-5,6-dihydroimidazo[1,2-α]-pyrazin-7(6H)-yl]-2-Oxoethylamin oder eines seiner pharmazeutisch verträglichen Salze mit Morphin kombiniert ist, für eine gleichzeitige, getrennte oder sich über einen bestimmten Zeitraum erstreckende therapeutische Verwendung bei der Behandlung oder Prävention von Schmerz.

3. Produkt nach Anspruch 2, **dadurch gekennzeichnet, dass** es aus der Kombination aus (1R)-1-[({(2R)-2-Amino-3-[(8S)-8-(cyclohexylmethyl)-2-phenyl-5,6-dihydroimidazo[1,2-α]pyrazin-7(8H)-yl]-3-oxopropyl}dithio)methyl]-2-[(8S)-8-(cyclohexylmethyl)-2-phenyl-5,6-dihydroimidazo[1,2-α]pyrazin-7(8H)-yl]-2-oxoethylamin oder einem seiner pharmazeutisch verträglichen Salze mit Morphin besteht, für eine gleichzeitig, getrennte oder sich über eine bestimmte Zeit erstreckende therapeutische Verwendung bei der Behandlung oder Prävention von Schmerz.

4. Produkt nach einem der Ansprüche 1 bis 3, **dadurch gekennzeichnet, dass** es für eine gleichzeitige Verwendung bestimmt ist.

5. Produkt nach einem der Ansprüche 1 bis 3, **dadurch gekennzeichnet, dass** es für eine getrennte Verwendung bestimmt ist.

6. Produkt nach einem der Ansprüche 1 bis 3, **dadurch gekennzeichnet, dass** es für eine über eine bestimmte Zeit verteilte Verwendung bestimmt ist.

7. Produkt nach einem der Ansprüche 1 bis 6, **dadurch gekennzeichnet, dass** der zu behandelnde Schmerz ausgewählt ist aus Schmerzen, die mit Krebs verbunden sind, Schmerzen, die mit anderen chronischen Erkrankungen als Krebs verbunden sind, neuropathischen Schmerzen, Schmerzen, die mit Radiculopathien verbunden sind, Adiposis dolorosa, Schmerzen, die mit Verbrennungen verbunden sind, Migräne, prä- und postoperativen Schmerzen, chronischen Schmerzen, Fibromyalgie, Algoneurodystrophie oder komplexe regionalem Schmerzsyndrom und zentralen Schmerzen als Folge von cerebral-vaskulären Schmerzen, Thalamusverletzungen und multipler Sklerose.

8. Produkt nach Anspruch 7, **dadurch gekennzeichnet, dass** der zu behandelnde Schmerz mit Krebs verbunden ist.

9. Produkt nach Anspruch 7, **dadurch gekennzeichnet, dass** der zu behandelnde Schmerz mit einher anderen chronischen Erkrankung als Krebs verbunden ist.
